(19) Europäisches Patentamt
European Patent Office
Office européen des brevets

(11) **EP 2 085 105 A1**

(12) **EUROPÄISCHE PATENTANMELDUNG**

(43) Veröffentlichungstag:
**05.08.2009 Patentblatt 2009/32**

(51) Int Cl.:
*A61M 15/00* (2006.01)

(21) Anmeldenummer: **09158952.3**

(22) Anmeldetag: **12.07.2001**

(84) Benannte Vertragsstaaten:
**AT BE CH CY DE DK ES FI FR GB GR IE IT LI LU MC NL PT SE TR**

(30) Priorität: **31.10.2000 DE 10053913**

(62) Dokumentnummer(n) der früheren Anmeldung(en) nach Art. 76 EPÜ:
**01116630.3 / 1 201 258**

(71) Anmelder: **Activaero GmbH**
**35285 Gemünden (DE)**

(72) Erfinder:
• **Scheuch, Gerhard**
 **35285, Gemünden (DE)**

• **Meyer, Thomas**
 **82343, Pöcking (DE)**
• **Müllinger, Bernhard**
 **84339, Unterdietfurt (DE)**
• **Brand, Peter**
 **82131, Gauting (DE)**

(74) Vertreter: **Peckmann, Ralf**
**Reinhard, Skuhra, Weise & Partner GbR**
**Patent- und Rechtsanwälte**
**Friedrichstrasse 31**
**80801 München (DE)**

Bemerkungen:
Diese Anmeldung ist am 28-04-2009 als Teilanmeldung zu der unter INID-Code 62 erwähnten Anmeldung eingereicht worden.

(54) **Verfahren und Apparatur zur Optimierung einer Dosisabscheidung bei einer inhalatorischen Medikamentenapplikation**

(57) Die Erfindung schafft ein Verfahren und eine Apparatur zur Optimierung einer Dosisabscheidung bei einer inhalatorischen Medikamentenapplikation. Das Verfahren besteht aus folgenden Schritten: Ermitteln eines individuellen Atemzugvolumens zur Optimierung der vorgesehenen Medikamentendosisabscheidung in einem vorbestimmten Lungenbereich, Anpassen der Medikamenten-Partikel-Konzentration in einer Medikamenten-Aerosol-Menge an das ermittelte Atemzugvolumen, sowie Erfassen und Signalisieren des Erreichens des zu inhalierenden Atemzugvolumens der Medikamenten-Aerosol-Menge, das für die vorgesehene Medikamentendosisabscheidung in dem vorbestimmten Lungenbereich mit einem Atemzug notwendig ist.

Fig. 1

EP 2 085 105 A1

**Beschreibung**

Die vorliegende Erfindung betrifft ein Verfahren und eine Apparatur zur Optimierung einer Dosisabscheidung bei einer inhalatorischen Medikamentenapplikation.

Das Inhalieren von Medikamenten nimmt immer mehr an Bedeutung zu, da systemisch wirkende Medikamente zunehmend über die Lunge appliziert werden. Dabei ist eine Optimierung der Dosisabscheidung von großer Bedeutung, da etliche Medikamente existieren oder neue Medikamente entwickelt werden, von denen einige mit einer hohen Dosis verabreicht werden müssen, eine geringe therapeutische Breite aufweisen, außerordentlich teuer sind und/oder bevorzugt in bronchialen oder alveolaren Lungenregionen zur Abscheidung gebracht werden müssen.

Für eine optimale Einsetzung dieser Medikamente ist es notwendig, mit möglichst wenigen Atemzügen zuverlässig eine hohe Medikamentendosis in bestimmten Lungenbereichen zur Abscheidung zu bringen und den Prozentsatz der abgeschiedenen Medikamentenpartikel zu maximieren.

Bisher wurde für eine Optimierung der Medikamentenabscheidung der Faktor Partikeleigenschaften der inhalierten Medikamenten-partikel, insbesondere die Partikelgröße, betrachtet. Das Inhalationsvolumen bleibt bei den bisherigen Optimierungsversuchen weitgehend ohne Berücksichtigung.

Die der vorliegenden Erfindung zugrundeliegende Problematik besteht also allgemein darin, eine Optimierung der Medikamentendosisabscheidung mittels einer Optimierung des Inhalationsvolumens zu schaffen.

Bei den bisher bekannten Ansätzen wird versucht, den Patienten ein optimales Atemmuster bzw. Atemmanöver anzutrainieren. Jedoch kehren die Patienten in der Regel bei längeren Inhalationszeiten zu ihrer spontanen Atmung mit einem relativ niedrigen Inhalationsvolumen zurück.

Außerdem wird bisher eine konstante Konzentration der Medikamenten-Partikel im Aerosolgemisch unabhängig von dem Inhalationsvolumen des einzelnen Patienten verwendet, ohne dass eine Abhängigkeit der Abscheidungswahrscheinlichkeit der Medikamentenpartikel von dem Inhalationsvolumen des einzelnen Patienten mit berücksichtigt wird. Bei einer tiefen Inhalation erhöht sich, wie bei Testversuchen festgestellt, die Abscheidungswahrscheinlichkeit der Medikamentenpartikel in der Lunge gegenüber einer Inhalation mit niedrigem Atemvolumen. Diesem Sachverhalt wird jedoch bisher nicht Rechnung getragen.

Außerdem unterscheiden sich die Atemzugvolumina eines einzelnen Patienten voneinander, wodurch es zu einer erheblichen Variation der in der Lunge ankommenden Medikamentendosis kommt. Zusätzlich ändert sich das Atemzugvolumen auch von Patient zu Patient.

In Anbetracht dessen liegt der Erfindung die Aufgabe zugrunde, ein Verfahren und eine Apparatur zur Optimierung einer Dosisabscheidung bei einer inhalatorischen Medikamentenapplikation zu schaffen, bei denen das individuelle Atemzugvolumen eines Patienten für eine Optimierung der Abscheidungswahrscheinlichkeit der Medikamentenpartikel in einem bestimmten Lungenbereich mit berücksichtigt wird.

Diese Aufgabe wird gelöst durch den Gegenstand des Anspruchs 1 bzw. den Gegenstand der Ausführungsform 1.

Die der vorliegenden Erfindung zugrundeliegende Idee besteht darin, zunächst ein individuelles Atemzugvolumen eines bestimmten Patienten mittels einer Ermittlungseinrichtung zur Optimierung der vorgesehenen Medikamentendosisabscheidung in einem vorbestimmten Lungenbereich zu ermitteln; danach die Medikamentenpartikel-Konzentration in der Medikamenten-Aerosol-Menge mittels einer Dosiereinrichtung an das ermittelte Atemzugvolumen anzupassen; und dann das Erreichen des zu inhalierenden Atemzugvolumens der Medikamenten-Aerosol-Menge, das für die vorgesehene Medikamentendosisabscheidung in dem vorbestimmten Lungenbereich mit einem Atemzug notwendig ist, mittels einer Erfassungseinrichtung zu erfassen und mittels einer Anzeigeeinrichtung zu signalisieren.

Das erfindungsgemäße Verfahren mit den Merkmalen des Anspruchs 1 und die entsprechende Apparatur gemäß Ausführungsform 1 weisen gegenüber den bekannten Lösungsansätzen den Vorteil auf, dass das individuelle Atemzugvolumen eines Patienten für eine Optimierung der Dosisabscheidung in einem bestimmten Lungenbereich mitberücksichtigt wird. Somit kann dem Tatbestand Rechnung getragen werden, dass die Wahrscheinlichkeit, mit der ein Medikamentenpartikel in der Lunge abgeschieden wird, im Wesentlichen von dem jeweiligen Inhalationsvolumen pro Atemzug abhängt. Bei einer Medikamentenapplikation in alveolaren Lungenbereichen ist ein möglichst hohes Inhalationsvolumen vorteilhaft. Dagegen ist bei Medikamentenapplikationen in bronchialen Lungenbereichen ein auf das maximale Atemzugvolumen des jeweiligen Patienten bezogenes reduziertes Inhalationsvolumen pro Atemzug für eine möglichst hohe Abscheidungswahrscheinlichkeit der Medikamentenpartikel in dem entsprechenden Lungenbereich vorteilhaft. Dabei inhaliert der Patient das Medikamenten-Aerosol nur in eine bestimmte Lungentiefe ein und atmet anschließend das Aerosol mit den nicht abgeschiedenen Medikamentenpartikel wieder aus.

Durch eine Bestimmung des Atemzugvolumens bzw. des inhalierten Volumens kann die Wahrscheinlichkeit der Medikamentendosis-abscheidung optimiert werden, wodurch die deponierte Medikamentenmenge maximiert und der Medikamentenausschuss minimiert wird. Die Ausbeute des deponierten Medikaments kann dadurch bis zu einem Faktor 3 erhöht werden, was insbesondere bei teuren Medikamenten von großem Vorteil ist. Außerdem verkürzt sich durch die höhere Ausbeute pro Atemzug die Inhalationszeit. Durch die Bestimmung des individuellen Atemmusters kann die in der Lunge applizierte Medikamentenmenge zusätzlich relativ genau bestimmt werden.

In den Unteransprüchen finden sich vorteilhafte Weiterbildungen und Verbesserungen des in Anspruch 1 angegebenen Verfahrens und der in Ausführungsform 1 angegebenen Apparatur.

Gemäß einer bevorzugten Weiterbildung kann das individuelle Atemzugvolumen, insbesondere das individuelle maximale Atemzugvolumen, mittels einer Lungenfunktions-Messeinrichtung bestimmt werden. Diese Messungen liefern genaue Werte über das individuelle Atemzugvolumen.

Gemäß einer weiteren bevorzugten Weiterbildung wird die Konzentration der Medikamentenpartikel in der Medikamenten-Aerosol-Menge und die abgeschiedene Medikamentendosis in der Lunge mittels eines Aerosolphotometers bestimmt. Aus der Anzahl der inhalierten Medikamentenpartikel und der Anzahl der ausgeatmeten Medikamentenpartikel können die in der Lunge abgeschiedenen Partikel ermittelt werden. Somit kann die jeweils bereits abgeschiedene Medikamentendosis ermittelt werden.

Gemäß einer weiteren bevorzugten Weiterbildung ist die Ermittlungseinrichtung über eine Kontrollereinrichtung mit der Dosiereinrichtung und/oder der Erfassungseinrichtung verbindbar. Somit kann ausgehend von einem ermittelten individuellen Atemzugvolumen eine bestimmte Konzentration der Medikamentenpartikel mittels der Dosiereinrichtung eingestellt und/oder der Erfassungseinrichtung ein bestimmtes zu inhalierendes Volumen vorgegeben werden, bei dessen Erreichen ein Signal ausgegeben wird.

Gemäß einer weiteren bevorzugten Weiterbildung ist die Dosiereinrichtung als Ultraschall-Vernebler, Vernebler mit vibrierender Düse oder als Pulver-Inhalator ausgebildet.

Gemäß einer weiteren bevorzugten Weiterbildung ist die Ermittlungseinrichtung und/oder die Erfassungseinrichtung als Flussmesseinrichtung, insbesondere als elektronische oder mechanische Flussmesseinrichtung, ausgebildet. Dies stellt eine technisch einfach zu realisierende und kostengünstige Volumenberechnungsmethode dar.

Gemäß einer weiteren bevorzugten Weiterbildung wird das maximale Atemzugvolumen und/oder das bereits inhalierte Volumen während des zu inhalierenden Atemzugs der Medikamenten-Aerosol-Menge mittels einer Differenzdruckmessung, Hitzdraht-Methode oder Ultraschall bestimmt. Dadurch kann zuverlässig und relativ genau das jeweilige Atemzugvolumen bestimmt und ein Erreichen des zu inhalierenden Volumens angezeigt werden.

Gemäß einer weiteren bevorzugten Weiterbildung ist das zu inhalierende Volumen mittels einer Begrenzungseinrichtung steuerbar. Somit kann aktiv ein bestimmtes zu inhalierendes Volumen vorgegeben werden, welches für eine optimale Dosisabscheidung in einem vorbestimmten Lungenbereich pro Atemzug inhaliert werden muss.

Gemäß einer weiteren bevorzugten Weiterbildung wird das Signalisieren des Erreichens des zu inhalierenden Atemzugvolumens der Medikamenten-Aerosol-Menge als akustisches Signal, insbesondere als Pfeiftonsignal, als mechanisches Signal, beispielsweise als Luftstoß, mechanischer Impuls oder als Vibration, oder als optisches Signal, insbesondere als Blink- oder Dauersignal, ausgebildet. Dadurch wird dem Patienten ein Erreichen des zu inhalierenden Volumens angezeigt, bei welchem die optimale Dosisabscheidung in dem vorbestimmten Lungenbereich erzielt worden ist.

Ausführungsbeispiele der Erfindung sind in den Zeichnungen dargestellt und in der nachfolgenden Beschreibung näher erläutert.

Es zeigen:

Fig. 1 eine schematische Darstellung einer Inhalationsapparatur gemäß einem ersten Ausführungsbeispiel der vorliegenden Erfindung;

Fig. 2 eine schematische Darstellung einer Inhalationsapparatur gemäß einem zweiten Ausführungsbeispiel der vorliegenden Erfindung; und

Fig. 3 eine schematische Darstellung einer Inhalationsapparatur gemäß einem dritten Ausführungsbeispiel der vorliegenden Erfindung.

In den Figuren bezeichnen gleiche Bezugszeichen gleiche oder funktionsgleiche Komponenten.

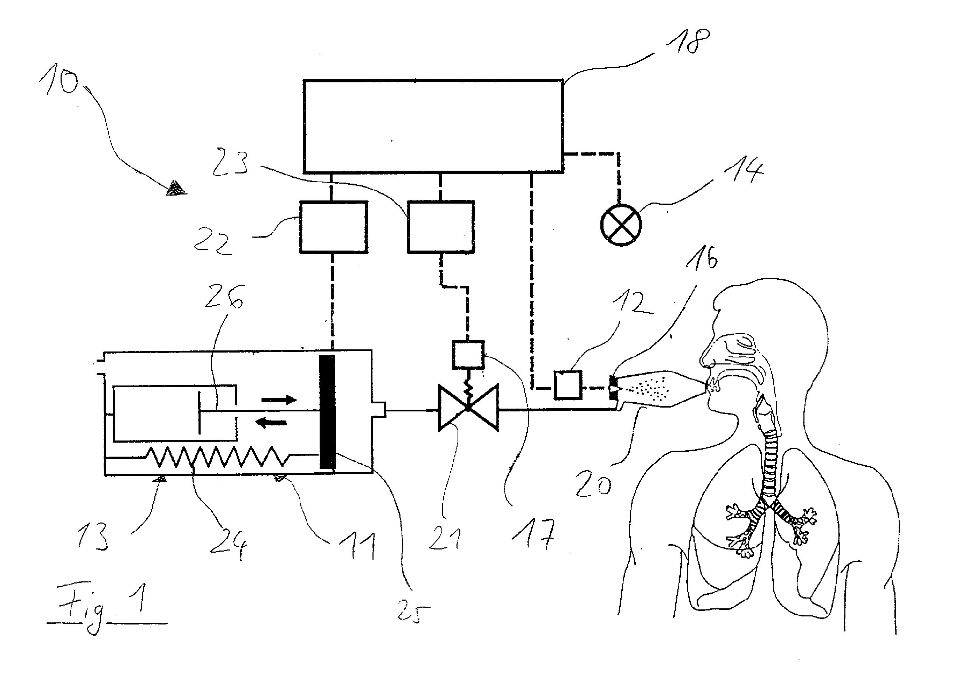

In Fig. 1 ist eine schematische Darstellung einer Inhalationsapparatur 10 gemäß einem ersten Ausführungsbeispiel der vorliegenden Erfindung dargestellt. Ein die zu inhalierende Medikamenten-Aerosol-Menge aufweisendes Mundstück 20 ist über ein Ventil 21 mit einer Ermittlungseinrichtung 11, die im vorliegenden Ausführungsbeispiel gleichzeitig als Erfassungseinrichtung 13 dient, verbunden. Das Ventil 21 ermöglicht einen Fluss in nur einer vorbestimmten Richtung zwischen dem Mundstück 20 und der Ermittlungseinrichtung 11 bzw. der Erfassungseinrichtung 13.

Die Ermittlungseinrichtung 11 bzw. die Erfassungseinrichtung 13 ist über einen Analog-Digital-Wandler 22 mit einer Kontrollereinrichtung 18, vorzugsweise einer Computereinheit 18, verbunden, in der als zentrale Steuerungseinrichtung vorgegebene Sollwerte vorteilhaft abgespeichert sind. Sie dient zur Steuerung der einzelnen Bauteile und zur Erfassung bestimmter Messwerte. Die Computereinheit 18 ist über einen Digital-AnalogWandler 23 mit einer Begrenzungseinrichtung 17 gekoppelt, die zur Steuerung des Ventils 21 vorgesehen ist.

Außerdem ist eine Dosiereinrichtung 12 an dem Mundstück 20 für eine Dosierung der Medikamentenpartikel-Konzentration angeordnet, die ebenfalls mit der Computereinheit 18 verbunden ist und die vor einem neuen Inhalationsvorgang für das entsprechende Medikamenten-Aerosol-Gemisch mit bestimmter Partikel-Konzentration in dem Mundstück 20 sorgt.

Ferner ist eine Anzeigeeinrichtung 14 zum Signalisieren eines Erreichens eines vorbestimmten Inhalationsvolumens oder eines Überschreitens einer bestimmten Flussgeschwindigkeit vorgesehen, die ebenfalls an einen Ausgang der Computereinheit 18 angeschlossen ist.

Zur Optimierung der Dosisabscheidung in einem vorbestimmten Lungenbereich mit einem Atemzug wird mittels der Inhalationsapparatur 10 gemäß dem ersten Ausführungsbeispiel der vorliegenden Erfindung wie folgt vorgegangen. Zunächst wird das maximale Atemzugvolumen des betreffenden Patienten ermittelt. Dabei bläst der Patient vorteilhaft sein maximales Atemzugvolumen in das Mundstück 20, wobei das Ventil 21 für einen Durchlass, und zwar als einzige Durchlassmöglichkeit, in Richtung der Ermittlungseinrichtung 11 geschaltet ist.

Um zu berechnen, mit welchem Inhalationsvolumen $V_{inh}$ ein Patient das Aerosol inhalieren kann, falls ein festes maximales Volumen $V_{max}$ durch die Erfassungseinrichtung 13 vorgegeben wird, wird folgende Berechnungsformel verwendet:

$$\text{für } V_{inh} < V_{Max} \qquad V_{inh} = (TLC - ITGV) \ast k_{Vol}$$

$$\text{für } V_{inh} >= V_{Max} \qquad V_{inh} = V_{Max}$$

Dabei sind die Parameter folgende:

ITGV:    intrathorakales Gasvolumen (1)
TLC:    totale Lungenkapazität (1)
$V_{inh}$:    Inhalationsvolumen (1)
$k_{Vol}$:    Volumenkoeffizient (<0.8 macht es dem Patienten einfach, das Inhalationsvolumen zu erreichen)
$V_{Max}$:    maximales Inhalationsvolumen (ca. 2 1)

Die Ermittlungseinrichtung 11 ist gemäß dem in Fig. 1 dargestellten ersten Ausführungsbeispiel der vorliegenden Erfindung als mechanische Flussmesseinrichtung in Form eines mit einem Federelement 24 vorgespannten Kolbens 25 ausgebildet. Dabei wird eine Änderung des mechanischen Kolbensystems mittels des Analog-Digital-Wandlers 22 als Signal der Computereinheit 18 zugeführt und mittels eines Programms in das vom Patienten ausgeatmete maximale Atemzugvolumen umgewandelt.

Um eine Medikamentenapplikation in alveolaren Lungenbereichen zu optimieren, ist das maximale Atemzugvolumen des Patienten die Ausgangsgröße für das bereitzustellende bzw. zu inhalierende Volumen der Medikamenten-Aerosol-Menge. Hingegen genügt für eine Optimierung der Medikamentenapplikation in bronchialen Lungenbereichen ein bestimmtes verringertes Inhalationsvolumen, das ausgehend vom gemessenen maximalen Inhalationsvolumen bestimmt werden kann. Dadurch kann für den vorbestimmten zu behandelnden Lungenbereich ein optimales Inhalationsvolumen pro Atemzug für den jeweiligen Patienten ermittelt werden.

Ausgehend von diesem zu inhalierenden Volumen wird über die Computereinheit 18 mittels der Dosiereinrichtung 12 die jeweilige optimale Medikamenten-Partikel-Konzentration eingestellt. Diese ist vorteilhaft durch vorherige Tests für bestimmte Atemzugvolumina festgelegt und in der Computereinheit abgespeichert.

Gleichzeitig wird durch die Computereinheit 18 das Ventil 21 derart umgeschaltet, dass nun ein Fluss von der Ermittlungseinrichtung 11 zum Mundstück 20 hin stattfinden kann. Die Ermittlungseinrichtung 11 dient nun vorteilhaft als Erfassungseinrichtung 13 für eine Erfassung des bereits inhalierten Volumens. Dabei kann die Begrenzungseinrichtung 17 als Regulator für das Ventil 21 dienen, wobei bei Erreichen des vorbestimmten zu inhalierenden Volumens das Ventil 21 geschlossen und somit der Fluss zum Mundstück 20 unterbrochen wird.

Die Erfassungseinrichtung 13 kann auch das zu inhalierende Volumen aktiv bereitstellen, d.h. die Erfassungseinrichtung 13 ist derart ausgebildet, dass gerade das zu inhalierende Volumen bereitgestellt wird. Zu schnelle Atemflüsse werden in diesem Ausführungsbeispiel durch die Dämpfung des Kolbens 25 mittels eines Dämpfungssystems 26 vermieden. Das Dämpfungssystem 26 bewegt sich mit einer Geschwindigkeit proportional zum Atemfluss, wobei ein bestimmter Weg des Systems gemessen wird, der proportional zum Volumenstrom und damit zum inhalierten Volumen ist. Somit wird das vom Patienten bereits inhalierte Volumen bestimmt und an die Computereinheit 18 übertragen. Bei Erreichen des vorgegebenen Volumens wird an der Anzeigeeinrichtung 14 ein für den Patienten erkennbares Signal ausgegeben, das als akustisches Signal, mechanisches Signal oder als optisches Signal ausgebildet werden kann.

Ebenfalls ist es denkbar, dass die Anzeigeeinrichtung 14 durch ein anderes ausgebildetes Signal dem Patienten nicht das Erreichen des zu inhalierenden Volumens anzeigt, sondern ein Überschreiten einer vorbestimmten Flussgeschwindigkeit signalisiert. Dabei dient die Computereinheit 18 als die zentrale Recheneinrichtung, welche die gemessenen Ist-Werte mit vorzugsweise abgespeicherten Soll-Werten vergleicht und demzufolge dem Patienten entsprechende Signale ausgibt bzw. das Inhalationsvolumen vorgibt und/oder die Flussgeschwindigkeit entsprechend begrenzt.

Als Kontrolle für die tatsächlich in dem vorbestimmten Lungenbereich deponierte Medikamentenmenge dient ein Aerosolphotometer 16, das vorzugsweise am Mundstück 20 angeordnet ist. Dabei wird das Aerosol durch das Aerosolphotometer 16 geleitet und mit beispielsweise einem Laser, einer Photodiode oder einem Weißlicht beleuchtet. Das Streulicht, das dadurch an den Aerosolpartikeln entsteht, kann über beispielsweise eine Photodiode oder einen Photomultiplier aufgenommen werden. Hieraus ergibt sich die Partikelkonzentration. Wird gleichzeitig das Inhalationsvolumen mit aufgezeichnet, kann daraus die in- und exhalierte Medikamenten-Aerosol-Menge festgestellt und die tatsächlich in der Lunge deponierte Medikamentenmenge quantisiert werden. Aus der errechneten deponierten Medikamentenmenge kann die Konzentration der Medikamentenpartikel mittels der Dosiereinrichtung 12, die vorteilhaft als Ultraschall-Vernebler, Vernebler mit vibrierender Düse oder als Pulver-Inhalator ausgebildet ist, gegebenenfalls nachgeregelt werden.

Es ist auch eine Berechnung der deponierten Medikamentenmenge $m_{med}$ möglich, dabei lautet die Berechnungsformel für Inhalationsapparate mit einem konstanten Medikamentenoutput:

$$m_{med} = n_{Atzg} \frac{Q_{Out} c_{Med}}{Q_{inh}} V_{inh}{}^2 k_{Dep}$$

Dabei sind die Parameter folgende:

$V_{inh}$: Inhalationsvolumen (1)
$m_{med}$: deponierte Medikamentenmenge (mg)
$n_{Atzg}$: Anzahl der Atemzüge
$Q_{Out}$: Volumenoutput des Inhalators (ml/min)
$Q_{inh}$: Inhalationsfluss (1/min)
$C_{med}$: Medikamentenkonzentration (mg/ml)
$k_{Dep}$: empirischer Depositionskoeffizient (ca. 0.325 bei Emphysematikern, bei Lungengesunden ca. 0.475)

Bei Inhalationsapparaten, die keinen konstanten Medikamentenoutput haben, berechnet sich die deponierte Medikamentenmenge $m_{med}$ wie folgt:

$$m_{med} = n_{Atzg}\, m_{Out}\, V_{inh}\, k_{Dep}$$

In beiden Fällen steigt die deponierte Medikamentenmenge $m_{med}$ mit zunehmendem Inhalationsvolumen $V_{inh.}$

Ausgehend von dem in Fig. 1 beschriebenen ersten Ausführungsbeispiel der vorliegenden Erfindung können verschiedene Prinzipien für eine Ermittlung eines individuellen Atemzugvolumens und eine Erfassung des Erreichens des zu inhalierenden Volumens realisiert werden.

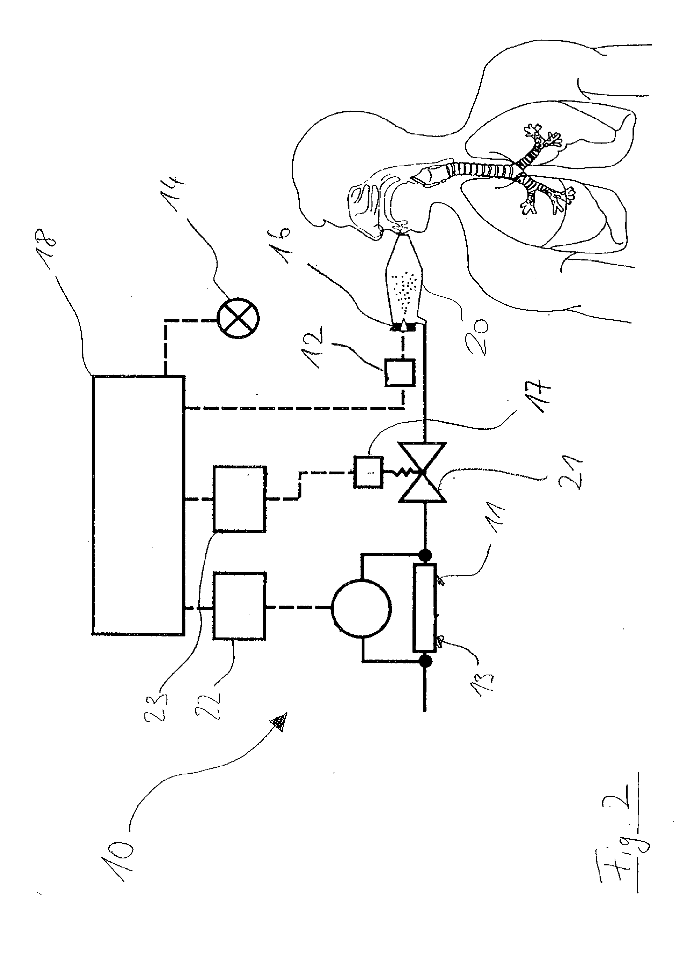

Fig. 2 zeigt eine Inhalationsapparatur 10 gemäß einem zweiten Ausführungsbeispiel der vorliegenden Erfindung. Dabei ist die Ermittlungseinrichtung 11 und die Erfassungseinrichtung 13 wiederum als eine gemeinsame Einrichtung ausgebildet, jedoch sind ebenso zwei einzelne Einrichtungen denkbar.

Im zweiten Ausführungsbeispiel wird das maximale Atemzugvolumen und das bereits inhalierte Volumen über eine Differenzdruckmessung zur Bestimmung des Durchflusses bestimmt. Bei der Durchflussmessung über den Differenzdruck wird der Atemfluss über einen Strömungswiderstand, vorzugsweise ein verengter Querschnitt, geführt. Es baut sich vor diesem Strömungswiderstand mit steigendem Durchfluss ein Wirkdruck auf, der proportional zum Durchfluss und damit zum inhalierten Volumen ist. Eine Umrechnung in das entsprechende Volumen wird aus der Flussgeschwindigkeit und der entsprechenden Zeitdauer realisiert. Die übrigen Bauteile bzw. der Ablauf des Verfahrens sind denen des ersten Ausführungsbeispiels analog und bedürfen keiner weiteren Erläuterung.

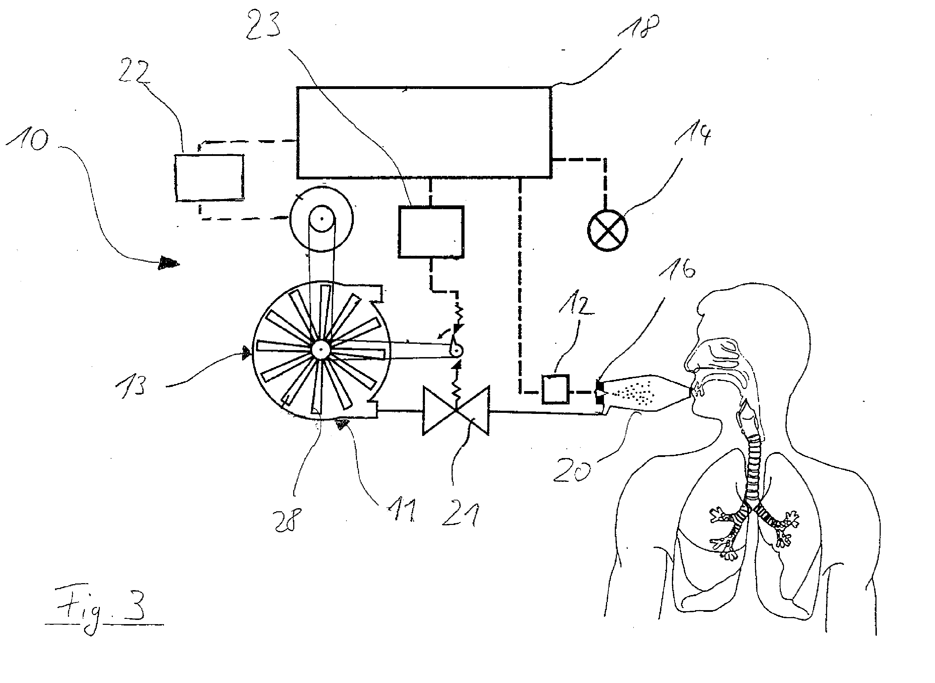

Fig. 3 zeigt eine Inhalationsapparatur 10 gemäß einem dritten Ausführungsbeispiel der vorliegenden Erfindung.

Dabei erfolgt die Volumenbestimmung mittels einer mechanischen Flussbestimmung über einen Rotor 28. Der Rotor 28 dreht sich proportional zum Durchfluss und somit zum inhalierten Volumen. Die übrigen Bauteile und Funktionsweisen bzw. das beschriebene Verfahren des ersten Ausführungsbeispiels sind analog anzuwenden und bedürfen keiner weiteren Ausführung.

Eine weitere Volumenmessmethode ist die Hitzdrahtmethode, bei der ein elektrisch beheizter Metalldraht, dessen Widerstand temperaturabhängig ist, in einen Gasstrom gebracht wird und von diesem abgekühlt wird. Die Wärmeabgabe ist dabei abhängig von der Geschwindigkeit des vorbeiströmenden Gases und dessen physikalischen Eigenschaften.

Eine Ultraschall-Strömungsmessung dient ebenfalls einer Volumenbestimmung. Dabei werden Schallwellen von beispielsweise einem piezo-elektrischen Schwinger erzeugt. Die Fortpflanzungsgeschwindigkeit dieser Schallwellen ändert sich in bewegten Medien mit der Strömungsgeschwindigkeit des Mediums. Die Richtung der Schallwellen darf dabei nicht 90° zur Flussrichtung betragen. Aus der Laufzeitverlängerung der Schallwellen über eine bestimmte Strecke kann somit die Flussgeschwindigkeit und damit über die Zeit das entsprechende Volumen ermittelt werden.

Das maximale Atemzugvolumen eines Patienten kann auch über Lungenfunktionsmessungen ausgeführt werden. Dabei wird die inspiratorische Lungenkapazität des Patienten gemessen, welche von dem Inhalationsvolumen nicht überschritten werden darf. Der Patient inhaliert beispielsweise 80% der inspiratorischen Lungenkapazität.

Durch eine mechanische oder elektronische Volumenbestimmung kann somit ein kostengünstiges Inhalationsgerät entwickelt werden, mit dem eine individuelle Inhalationsdosimetrie möglich ist. Dabei kann die Dosisabscheidung in einem vorbestimmten Lungenbereich durch eine Optimierung des entsprechenden Inhalationsvolumens optimiert werden. Medikamente mit geringer therapeutischer Breite können damit mit weniger Risiko und teure Medikamente ökonomischer eingesetzt werden.

Das im Vorstehenden beschriebene Verfahren und die entsprechende Inhalationsapparatur werden insbesondere zum Einbringen von pharmazeutisch wirksamen Substanzen bzw. Wirkstoffen in die Lunge verwendet. Der Wirkstoff, wie hier beschrieben, schließt Wirkstoffe, Medikamente, Verbindungen, Zusammensetzungen oder Mischungen von Stoffen ein, die einen pharmakologischen, - oft vorteilhaften - Effekt erzielen. Dies schließt Speisen, Nahrungsergänzungsstoffe, Nährstoffe, Medikamente, Impfstoffe, Vitamine und weitere nützliche Wirkstoffe mit ein.

Die Ausdrücke, wie sie hier benutzt werden, schließen weiterhin alle physiologisch oder pharmakologisch aktiven Substanzen ein, die einen lokalen oder systemischen Effekt in einem Patienten bewirken. Der aktive Wirkstoff, der zugeführt werden kann, schließt Antikörper, antivirale Wirkstoffe, Antiepileptika, Analgetika, entzündungshemmende Wirkstoffe und Bronchodilatatoren ein und kann eine organische oder anorganische Verbindung sein, die ohne Beschränkungen auch Medikamente einschließt, die auf das periphere Nervensystem, adrenerge Rezeptoren, cholinerge Rezeptoren, Skelettmuskulatur, kardiovaskuläres System, glatte Muskulatur, Blutkreislaufsystem, neuronale Verbindungen, endokrines- und Hormonsystem, Immunsystem, reproduktives System, Skelettsystem, Nahrungszufuhr- und exkretorisches System, Histaminkaskade oder zentrales Nervensystem wirken. Geeignete Wirkstoffe können z.B. aus Polysacchariden, Steroiden, Hypnotika und Sedativa, antriebssteigernden Mitteln, Beruhigungsmitteln, krampflösenden und muskelentspannenden Mitteln, Antiparkinson-Substanzen, Analgetica, Entzündungshemmern, antimikrobiellen Wirkstoffen, Antimalariamitteln, Hormonen inklusive empfängnisverhütenden Mitteln, Syphaticominetica, Polypeptiden und Proteinen, die physiologische Effekte hervorrufen, Diuretica, Fettstoffwechsel regulierenden Substanzen, antiandrogenen Wirkstoffen, gegen Parasiten gerichtete Mittel, neoplastisch und antineoplastisch wirkenden Stoffen, Antidiabetika, Nahrungs- und Nahrungsergänzungsstoffen, wachstumsfördernden Stoffen, Fetten, stuhlregulierenden Stoffen, Elektrolyten, Impfstoffen und Diagnostika bestehen.

Das Verfahren und die Inhalationsapparatur eignen sich besonders zur inhalativen Applikation von verschiedenen Wirkstoffen (ist aber nicht darauf beschränkt) wie:

Insulin, Calcitonin, Erythropoietin (EPO), Faktor VIII, Faktor IX, Cyclosporin, Granulozyte Colony Stimulating Factor (GCSF), Alpha-1 Proteinase Inhibitor, Elcatonin, Granulocyte Makrophage Colony Stimulating Factor (GMCSF), Wachstumshormone, menschliches Wachstumshormon (HGH), Growth hormon releasing hormone (GHRH), Heparin, niedermolekulares Heparin (LMWH), Interferon alpha, Interferon beta, Interferon gamma, Interleukin-2, Luteinizing hormone releasing hormone (LHRH), Somatostatin, Somatostatin-Analoge einschließlich Octreotide, Vasopressinanaloge, Follikel stimulierendes Hormon (FSH), Insulinlike growth factor, Insulintropin, Interleukin-I Rezeptorantagonist, Interleukin-3, Interleukin-4, Interleukin-6, Macrophage colony stimulating Factor (M-CSF), Nervenwachstumsfaktor, Parathyroidhormon (PTH), Thymosin alpha 1, IIb/IIIa Inhibitor, Alpha-1 Antitrypsin, Antikörper gegen respiratorisch syncytischen Virus, Cystic fibrosis transmembrane regulator gene (CFTR), Desoxyribonuclease (Dnase), Bactericide, permeability increasing protein (BPI), anti-CMV Antikörper, Interleukin-1 Rezeptor, Retinol-Säuren, Pentamidine, Albuterolsulfat, Metaproterenolsulfat, Beclomethasondiprepionat, Triamcinolonacetamid, Budesonidacetonide, Ipratropiumbromid, Flunisolide, Fluticasone, Cromolynsodium, Ergotamintartrat und die Analogen, Agonisten und Antagonisten der oben genannten Stoffe. Aktive Wirkstoffe können weiterhin Nukleinsäuren in Form von reinen Nukleinsäuremolekülen, viralen Vektoren, assoziierten viralen Partikeln, Nukleinsäuren, die mit Lipiden oder einem Lipide beinhaltenden Material assoziiert oder darin enthalten sind, Plasmid-DNA oder -RNA oder andere Konstrukte aus Nukleinsäuren sein, die geeignet sind für die Zell-Transfection oder -Transformation, besonders bei Zellen der alveolären Region der Lunge. Der aktive Wirkstoff kann in verschiedenen Formen vorliegen, wie lösliche oder unlösliche geladene oder ungeladene Moleküle, Komponenten molekularer Komplexe oder pharmakologisch akzeptierte Hilfsstoffe. Der aktive Wirkstoff kann bestehen aus natürlich vorkommenden Molekülen, oder deren rekombinanter Produktion, oder die Moleküle können Analoge der natürlich vorkommenden oder rekombinant erzeugten aktiven Wirkstoffe sein, bei denen eine oder mehrere Aminosäuren addiert oder entfernt wurden. Weiterhin kann der aktive Wirkstoff abgeschwächten Lebendimpfstoff oder abgetötete Viren für den Impfstoffgebrauch enthalten. Im Falle des Wirkstoffes Insulin sind natürlich extrahiertes menschliches Insulin, rekombinantes menschliches Insulin, aus Rindern und/oder Schweinen extrahiertes Insulin, rekombinantes Schweine- oder Rinderinsulin und Mischungen der oben genannten Insuline eingeschlossen. Das Insulin kann in reiner, also in substanziell gereinigter Form vorliegen, kann aber auch Auszüge wie kommerziell üblich enthalten. Im Ausdruck "Insulin" sind auch Insulin-Analoge enthalten, bei denen eine oder mehrere der Aminosäuren des natürlich vorkommenden oder rekombinanten Insulins addiert oder entfernt wurden.

Bevorzugte Ausführungsformen der Erfindung

1. Apparatur (10) zur Optimierung einer Dosisabscheidung bei einer inhalatorischen Medikamentenapplikation mit:

einer Ermittlungseinrichtung (11) zum Ermitteln eines individuellen Atemzugvolumens zur Optimierung der vorgesehenen Medikamentendosis-abscheidung in einem vorbestimmten Lungenbereich;

einer Dosiereinrichtung (12) zum Anpassen der Medikamenten-Partikel-Konzentration in einer Medikamenten-Aerosol-Menge an das ermittelte Atemzugvolumen; und mit

einer Erfassungseinrichtung (13) zum Erfassen und einer Anzeigeeinrichtung (14) zum Signalisieren des Erreichens des zu inhalierenden Atemzugvolumens der Medikamenten-Aerosol-Menge, das für die vorgesehene Medikamentendosis-abscheidung in dem vorbestimmten Lungenbereich mit einem Atemzug notwendig ist.

2. Apparatur nach Ausführungsform 1, **dadurch gekennzeichnet**, **dass** die Ermittlungseinrichtung (11) als Lungenfunktions-messeinrichtung ausgebildet ist.

3. Apparatur nach einer der Ausführungsformen 1 oder 2, da**durch gekennzeichnet, dass** die E rmittlungseinrichtung (11) über eine Kontrollereinrichtung (18) mit der Dosiereinrichtung (12) und/oder der Erfassungseinrichtung (13) verbindbar ist.

4. Apparatur nach einer der Ausführungsformen 1 bis 3, **dadurch gekennzeichnet, dass** die Dosiereinrichtung (12) als Ultraschall-Vernebler, Vernebler mit vibrierender Düse oder als Pulver-Inhalator ausgebildet ist.

5. Apparatur nach einer der Ausführungsformen 1 bis 4, **gekennzeichnet durch** ein Aerosolphotometer (16) zur Bestimmung der applizierten Medikamentendosis.

6. Apparatur nach einer der Ausführungsformen 1 bis 5, **dadurch gekennzeichnet, dass** die Ermittlungseinrichtung (11) und/oder die Erfassungseinrichtung (13) als Flussmesseinrichtung, insbesondere als elektronische oder mechanische Flussmesseinrichtung, ausgebildet ist.

7. Apparatur nach einer der Ausführungsformen 1 bis 6, **dadurch gekennzeichnet, dass** das zu inhalierende Volumen mittels einer Begrenzungseinrichtung (17) steuerbar ist.

8. Apparatur nach einer der Ausführungsformen 1 bis 7, **dadurch gekennzeichnet, dass** die Anzeigeeinrichtung (14) als akustische Anzeigeeinrichtung ausgebildet ist.

9. Apparatur nach einer der Ausführungsformen 1 bis 8, da**durch gekennzeichnet, dass** die Anzeigeeinrichtung (14) als mechanische Anzeigeeinrichtung, beispielsweise einer Luftstoßeinrichtung, einer mechanischen Impulseinrichtung oder einer Vibratoreinrichtung, ausgebildet ist.

10. Apparatur nach einer der Ausführungsformen 1 bis 9, da**durch gekennzeichnet, dass** die Anzeigeeinrichtung (14) als optische Anzeigeeinrichtung, insbesondere als Blink- oder Dauerleuchteinrichtung, ausgebildet ist.

11. Verwendung des Verfahrens nach einem der Ansprüche 1 bis 11 und der Apparatur nach einer der Ausführungsformen 1 bis 10 zum Einbringen von pharmazeutisch wirksamen Substanzen in die Lunge.

12. Verwendung der Apparatur nach einer der Ausführungsformen 1 bis 10 zum Einbringen von pharmazeutisch wirksamen Substanzen in die Lunge.

**Bezugszeichenliste**

| | |
|---|---|
| 10 | Inhalationsapparatur |
| 11 | Ermittlungseinrichtung |
| 12 | Dosiereinrichtung |
| 13 | Erfassungseinrichtung |
| 14 | Anzeigeneinrichtung |
| 16 | Aerosolphotometer |
| 17 | Begrenzungseinrichtung |
| 18 | Kontrollereinrichtung |
| 20 | Mundstück |
| 21 | Ventil |
| 22 | A/D-Wandler |
| 23 | D/A-Wandler |
| 24 | Federelement |
| 25 | Kolben |
| 26 | Dämpfungssystem |
| 28 | Rotor |

**Patentansprüche**

1. Verfahren zur Optimierung einer Dosisabscheidung bei einer inhalatorischen Medikamentenapplikation mittels einer Inhalationsapparatur (10), bestehend aus folgenden Schritten:

   Ermitteln eines individuellen Atemzugvolumens zur Optimierung der vorgesehenen Medikamentendosisabscheidung in einem vorbestimmten Lungenbereich;
   Anpassen der Medikamenten-Partikel-Konzentration in einer Medikamenten-Aerosol-Menge an das ermittelte Atemzugvolumen; und
   Erfassen und Signalisieren des Erreichens des zu inhalierenden Atemzugvolumens der Medikamenten-Aerosol-Menge, das für die vorgesehene Medikamentendosisabscheidung in dem vorbestimmten Lungenbereich mit einem Atemzug notwendig ist.

2. Verfahren nach Anspruch 1, **dadurch gekennzeichnet, dass** das maximale Atemzugvolumen individuell ermittelt wird.

3. Verfahren nach einem der Ansprüche 1 oder 2, **dadurch gekennzeichnet, dass** das individuelle Atemzugvolumen durch Lungenfunktionsmessungen bestimmt wird.

4. Verfahren nach einem der vorhergehenden Ansprüche, **dadurch gekennzeichnet, dass** die Konzentration der Medikamenten-Partikel in der Medikamenten-Aerosol-Menge mittels einem Aerosolphotometer (16) bestimmt wird.

5. Verfahren nach einem der vorhergehenden Ansprüche, **dadurch gekennzeichnet, dass** das individuelle Atemzugvolumen und/oder das inhalierte Volumen während des zu inhalierenden Atemzugs der Medikamenten-Aerosol-Menge über eine Flussmessung, insbesondere eine elektronische oder mechanische Flussmessung, bestimmt wird.

6. Verfahren nach einem der vorhergehenden Ansprüche, **dadurch gekennzeichnet, dass** das individuelle Atemzugvolumen und/oder das inhalierte Volumen während des zu inhalierenden Atemzugs der Medikamenten-Aerosol-Menge mittels einer Differenzdruckmessung, Hitzdraht-Methode oder Ultraschall bestimmt wird.

7. Verfahren nach einem der vorhergehenden Ansprüche, **dadurch gekennzeichnet, dass** das zu inhalierende Atemzugvolumen aktiv zur Verfügung gestellt wird.

8. Verfahren nach einem der vorhergehenden Ansprüche, **dadurch gekennzeichnet, dass** das Signalisieren des Erreichens des zu inhalierenden Atemzugvolumens der Medikamenten-Aerosol-Menge als akustisches Signal, insbesondere als Pfeiftonsignal, ausgebildet wird.

9. Verfahren nach einem der vorhergehenden Ansprüche, **dadurch gekennzeichnet, dass** das Signalisieren des Erreichens des zu inhalierenden Atemzugvolumens der Medikamenten-Aerosol-Menge als mechanisches Signal, beispielsweise als Luftstoß, mechanischer Impuls oder als Vibration, ausgebildet wird.

10. Verfahren nach einem der vorhergehenden Ansprüche, **dadurch gekennzeichnet, dass** das Signalisieren des Erreichens des zu inhalierenden Atemzugvolumens der Medikamenten-Aerosol-Menge als optisches Signal, insbesondere als Blink- oder Dauersignal, ausgebildet wird.

11. Verfahren nach einem der vorhergehenden Ansprüche, **dadurch gekennzeichnet, dass** die abgeschiedene Medikamentendosis mittels einem Aerosolphotometer (16) bestimmt wird.

Fig. 1

EP 2 085 105 A1

Fig. 2

Fig. 3

**Europäisches Patentamt**
**European Patent Office**
**Office européen des brevets**

**ERKLÄRUNG**

die nach Regel 63 des Europäischen Patent-übereinkommens für das weitere Verfahren als europäischer Recherchenbericht gilt

**Nummer der Anmeldung**

EP 09 15 8952

| Die Recherchenabteilung ist der Auffassung, daß die vorliegende Patentanmeldung den Vorschriften des EPÜ in einem solchen Umfang nicht entspricht, daß sinnvolle Ermittlungen über den Stand der Technik auf der Grundlage aller Patentansprüche nicht möglich sind.<br><br>Grund:<br><br>Eine sinnvolle Recherche auf der Grundlage aller Ansprüche ist nicht möglich, da diese sich beziehen auf - Verfahren zur therapeutischen Behandlung des menschlichen oder tierischen Körpers - Artikel 53 (c) EPÜ<br><br>Der Anmelder wird darauf hingewiesen, dass im Zuge der Prüfung eine Recherche durchgeführt werden kann, sollten die einer Erklärung gemäss Regel 63 EPÜ zugrundeliegenden Mängel behoben worden sein (Vgl. EPA-Richtlinien C-VI, 8.2).<br>----- | **KLASSIFIKATION DER ANMELDUNG (IPC)**<br><br>INV.<br>A61M15/00 |

EPO FORM 1504 (P04F39)

| Recherchenort | Abschlußdatum | Prüfer |
|---|---|---|
| München | 22. Juni 2009 | Mausser, Thomas |